# EUROPÄISCHE PATENTSCHRIFT

(11) **EP 2 413 859 B1**
(45) Veröffentlichungstag und Bekanntmachung des Hinweises auf die Patenterteilung: **30.01.2013**
(21) Anmeldenummer: 10710556.1
(22) Anmeldetag: 24.03.2010
(51) Int. Cl.: A61F 13/15

(54) **VERFAHREN ZUM HERSTELLEN EINER ABSORBIERENDEN INKONTINENZWEGWERFWINDEL UND VORRICHTUNG ZUR DURCHFÜHRUNG DES VERFAHRENS**
METHOD FOR PRODUCING AN ABSORBENT DISPOSABLE INCONTINENCE DIAPER AND APPARATUS FOR CONDUCTING THE METHOD
PROCÉDÉ DE FABRICATION D'UNE COUCHE D'INCONTINENCE, JETABLE ET ABSORBANTE, ET DISPOSITIF POUR LA MISE EN UVRE DU PROCÉDÉ

(30) Priorität: 04.04.2009 DE 102009016381
(43) Veröffentlichungstag der Anmeldung: 08.02.2012
(73) Patentinhaber: Paul Hartmann Aktiengesellschaft, 89522 Heidenheim (DE)
(72) Erfinder: KNECHT, Theresia, 73433 Aalen (DE)
(74) Vertreter: Dreiss
(86) Internationale Anmeldenummer: PCT/EP2010/001827
(87) Internationale Veröffentlichungsnummer: WO 2010/112160

(56) Entgegenhaltungen:
- EP-A1- 2 020 215
- WO-A1-2006/007226
- WO-A1-2009/002235
- WO-A2-2008/155618

## Beschreibung

Die Erfindung betrifft ein Verfahren zum Herstellen einer absorbierenden Inkontinenzwegwerfwindel, mit einem einen Saugkörper aufweisenden Hauptteil, umfassend einen Vorderbereich mit vorderen seitlichen Längsrändern, einen Rückenbereich mit hinteren seitlichen Längsrändern und einen dazwischen angeordneten, zwischen den Beinen eines Benutzers zu liegen kommenden Schrittbereich, und mit beidseits an den Rückenbereich angefügten hinteren Seitenabschnitten und beidseits an der Vorderbereich angefügten vorderen Seitenabschnitten, welche sich in einer Querrichtung der Inkontinenzwegwerfwindel über die seitlichen vorderen bzw. hinteren Längsränder des Hauptteils hinaus erstrecken und in einer Längsrichtung der Inkontinenzwegwerfwindel voneinander beabstandet sind, wobei die hinteren und vorderen Seitenabschnitte zum Anlegen der Inkontinenzwegwerfwindel miteinander lösbar verbindbar sind.

Bei derartigen Inkontinenzwegwerfwindeln sind die erwähnten Seitenabschnitte häufig aus einem anderen Material gebildet als der Hauptteil. Beispielsweise können die Seitenabschnitte, die häufig auch als "Ohren" der Inkontinenzwegwerfwindel bezeichnet werden, atmungsaktiv, insbesondere luft- und/oder wasserdampfdurchlässig ausgebildet werden, wohingegen der Hauptteil der häufig auch als Chassis bezeichnet wird, flüssigkeitsundurchlässig ausgeführt sein kann. Zum Schließen der Inkontinenzwegwerfwindel werden die vorzugsweise unlösbar am Rückenbereich angefügten Seitenabschnitte auf die Bauchseite des Benutzers geschlagen und dort entweder mit der Außenseite des Vorderbereichs des Hauptteils oder mit der Außenseite der Seitenabschnitte des Vorderbereichs lösbar verbunden.

Es wäre beispielsweise denkbar, dass die vorderen und hinteren Seitenabschnitte von im Wesentlichen rechteckigen Abschnitten eines Flachmaterials gebildet werden, die dann getaktet in einem sogenannten cut-and-place-Verfahren an eine die Windelhauptteile bildende Hauptteilbahn appliziert werden. Solchenfalls werden die Beinausschnitte, die von den einander zugewandten Längsrändern der hinteren und vorderen Seitenabschnitte und von einem insbesondere sanduhrförmig konturierten Hauptteil begrenzt werden, ebenfalls mit quer zur Maschinenrichtung, also quer zur Längsrichtung der Inkontinenzwegwerfwindel, erstreckten geraden Rändern begrenzt. Demgegenüber ist es aus verschiedenen Gründen wünschenswert, konturierte Beinöffnungsbereiche bei der Inkontinenzwegwerfwindel vorzusehen, worunter nachfolgend verstanden wird, dass der die Beinöffnungen begrenzende Rand des hinteren Seitenabschnitts und/oder des vorderen Seitenabschnitts nicht exakt in Querrichtung, also nicht senkrecht zur Windellängsrichtung verläuft. Er könnte beispielsweise zwar gerade, jedoch schräg zur Längsrichtung der Inkontinenzwegwerfwindel verlaufende Abschnitte und/oder kurvenförmige Abschnitte umfassen. In einer bevorzugten Ausführungsform der Inkontinenzwegwerfwindel weist der die Beinöffnungen begrenzende Rand der Seitenabschnitte ausschließlich kurvenförmige, also gekrümmt verlaufende Abschnitte auf. Der minimale Kurvenradius beträgt dabei vorzugsweise mindestens 5 mm, insbesondere bevorzugt mindestens 10 mm. Vorzugsweise umfasst die Kontur der Beinöffnungsbereiche kurvenförmige Abschnitte mit variierendem Kurvenradius.

Ein Verfahren zum Herstellen derartiger Inkontinenzwegwerfwindeln des sogenannten offenen Typs ist aus EP 07 015 141.0 (bitte Publikationsnummer einfügen) der Anmelderin bekannt.

Der vorliegenden Erfindung liegt die Aufgabe zugrunde, ein Verfahren der eingangs genannten Art anzugeben, das in prozesstechnisch günstiger Weise und im Hinblick auf den dabei auftretenden Schnittabfall möglichst wirtschaftlich durchführbar ist.

Diese Aufgabe wird bei einem Verfahren der genannten Art erfindungsgemäß dadurch gelöst, dass zur Konturierung von beidseitigen Beinöffnungsbereichen der Inkontinenzwegwerfwindel die hinteren Seitenabschnitte und/oder die vorderen Seitenabschnitte durch einen Abtrennvorgang von endlos zugeführten Seitenabschnittbahnen gebildet und dabei konturiert werden, und dass eine endlos zugeführte Hauptteilbahn vor dem Anfügen der endlos zugeführten Seitenabschnittbahnen quer zur Bahnebene und quer zur Maschinenrichtung eingefaltet wird, dass dann die Seitenabschnittbahnen an die Hauptteilbahn angefügt werden, dass der die Seitenabschnittbahnen erfassende Abtrennvorgang beidseits ausgeführt wird, so dass dann der Verbund aus Hauptteilbahn und angefügten Seitenabschnittbahnen in Maschinenrichtung beschleunigt und dabei entfaltet werden kann, wobei die vorderen und hinteren Seitenabschnitte in der Längsrichtung voneinander beabstandet werden können.

Dadurch, dass die Seitenabschnittbahnen, welche die hinteren und/oder vorderen Seitenabschnitte bilden, endlos zugeführt werden und bereits beim Ablängen der betreffenden Seitenabschnitte von diesen endlosen Seitenabschnittbahnen die Konturierung der einander zugewandten Ränder der Seitenabschnitte realisiert wird, lässt sich eine prozesstechnisch günstige und stabile Verfahrensführung realisieren, weil mit endlosen Materialbahnen bis zu deren Anfügen an die Hauptteilbahn gearbeitet werden kann. Durch die weitere erfindungsgemäße Maßnahme des Einfaltens der Hauptteilbahn aus der Bahnebene in eine gewissermaßen inaktive Zwischenspeicherstellung können die beidseitigen Seitenabschnittbahnen endlos zugeführt und endlos an die Hauptteilbahn angefügt werden, mit der Folge, dass der Schnittabfall im Wesentlichen wenigstens nahezu auf das zwingend zum Konturieren der Ränder der Seitenabschnitte bzw. der Beinöffnungsbereiche der herzustellenden Windel erforderliche Maß reduziert wird.

Bei einer bevorzugten Verfahrensführung wird also die Hauptteilbahn zugeführt und dann aus der Bahnebene eingefaltet, sodann werden beidseits die Seitenabschnittbahnen endlos zugeführt und an die Hauptteilbahn angefügt. Vorzugsweise danach erfolgt der die Seitenabschnittbahnen erfassende Abtrennvorgang, der die Funktion eines Formschnitts zur Konturierung der einander zugewandten Ränder der Seitenabschnitte bzw. wenigstens eines hinteren oder vorderen Seitenabschnitts zur Bildung konturierter Beinöffnungsbereiche hat. Danach kann der Verbund aus Hauptteilbahn und angefügten und konturierten Seitenabschnitten in der Maschinenrichtung beschleunigt und dabei entfaltet werden. Dieser Vorgang des Beschleunigens und Entfaltens dürfte sich in der überwiegenden Anzahl von Anwendungen im Hinblick auf nachfolgende Konfigurationsschritte als vorteilhaft erweisen; es wäre aber zumindest grundsätzlich denkbar, dass die erwähnte Einfaltung der Hauptteilbahn verbleibt und eine erste Faltung des Produkts für Verpackungszwecke darstellt. - Wenn vorstehend von der Zuführung und Einfaltung der Hauptteilbahn die Rede ist, so kann sich dies auch auf eine Komponente einer den späteren Hauptteil bildenden endlosen Bahn, wie z.B. eine endlose Topsheetbahn oder eine endlose Backsheetbahn, beziehen, die dann nach der erfindungsgemäßen Verfahrensführung mit weiteren den Hauptteil bildenden Komponenten, wie Saugkörper, Verteilerschicht, Topsheet(bahn), Backsheet(bahn), etc., verbunden wird.

Nach einer ersten Ausführungsform des erfindungsgemäßen Verfahrens werden sowohl die hinteren als auch die vorderen Seitenabschnitte beidseits von derselben Seitenabschnittbahn gebildet. Solchenfalls erweist es sich als vorteilhaft, dass die Tiefe der Einfaltung der Hauptteilbahn derart ist, dass die in der Bahnebene zum Anfügen der Seitenabschnittbahnen zur Verfügung stehende Länge der Hauptteilbahn je Inkontinenzwegwerfwindel der Länge der Seitenabschnitte je Inkontinenzwegwerfwindel entspricht (Anspruch 2). Die Einfalttiefe beträgt solchenfalls 0,5 x (Produktlänge - Seitenabschnittlänge), wobei die Seitenabschnittlänge die Summe der Längserstreckung des hinteren und vorderen Seitenabschnitts in Maschinenrichtung bezeichnet. Auf diese Weise wird gewissermaßen der gesamte nur von der Hauptteilbahn gebildete Schrittbereich der Hauptteilbahn weggefaltet, so dass die Seitenabschnittbahnen in optimal wirtschaftlicher Weise mit minimiertem Schnittabfall materialtechnisch ausgenutzt werden können. Nach dem Anfügen der Seitenabschnittbahnen und der Ausführung des die Seitenabschnittbahnen erfassenden Abtrennvorgangs, d. h. des Formschnitts zur Konturierung der einander zugewandten Ränder der Seitenabschnitte, kann dann der Verbund aus Hauptteilbahn und Seitenabschnittbahnen wieder entfaltet werden.

Nach einer weiteren Ausführungsform des erfindungsgemäßen Verfahrens können die jeweiligen vorderen und hinteren Seitenabschnitte von verschiedenen endlos zugeführten Seitenabschnittbahnen gebildet werden, d. h. sie können auch unterschiedliche Materialien umfassen. Hierfür gibt es zwei Varianten:

Nach der ersten Variante wird in einem ersten Applikationsschritt beidseits eine erste Seitenabschnittbahn an die zuvor eingefaltete Hauptteilbahn angefügt, und daran anschließend wird ein erster die ersten Seitenabschnittbahnen erfassender Abtrennvorgang beidseits ausgeführt und die Hauptteilbahn wieder entfaltet. Sodann wird in einem zweiten Applikationsschritt beidseits eine zweite Seitenabschnittbahn an die zuvor erneut eingefaltete Hauptteilbahn angefügt und daran anschließend ein zweiter die zweiten Seitenabschnittbahnen erfassender Abtrennvorgang beidseits ausgeführt (Anspruch 3). Die Einfaltung der Hauptteilbahn während des ersten Applikationsschritts und die Einfaltung des Verbunds aus Hauptteilbahn und ersten Seitenabschnitten in dem zweiten Applikationsschritt ist dabei größer als im ersten Ausführungsbeispiel, wo die Hauptteilbahn lediglich über die Längserstreckung des Schrittbereichs in eine inaktive Zwischenspeicherstellung eingefaltet wurde. Im vorliegenden Fall dieser zweiten Ausführungsform des erfindungsgemäßen Verfahrens erweist es sich wiederum als vorteilhaft, wenn die Einfaltung bzw. die Einfalttiefe derart maximal gewählt wird, dass das Material der betreffenden Seitenabschnittbahnen maximal, d. h. bis auf den zwingenden zur Konturierung der Beinausschnitte erforderlichen Schnittabfall in Maschinenlängsrichtung ausgenutzt werden kann.

Nach der zweiten Variante, bei der wiederum die jeweiligen vorderen und hinteren Seitenabschnitte von verschiedenen Seitenabschnittbahnen gebildet werden, wird in einem ersten Applikationsschritt beidseits eine erste Seitenabschnittbahn an eine zuvor eingefaltete erste Komponente der Hauptteilbahn, beispielsweise an eine endlose Backsheet-Bahn, angefügt und daran anschließend ein erster die ersten Seitenabschnittbahnen erfassender Abtrennvorgang beidseits ausgeführt und die Hauptteilbahn wieder entfaltet. In einem zweiten, insbesondere parallel zu dem ersten ablaufenden Applikationsschritt wird beidseits eine zweite Seitenabschnittsbahn an eine zuvor eingefaltete zweite Komponente der Hauptteilbahn, beispielsweise an eine endlose Topsheet-Bahn, angefügt und daran anschließend wird ein zweiter die zweiten Seitenabschnittbahnen erfassender Abtrennvorgang beidseits ausgeführt und die Hauptteilbahn wieder entfaltet. Die erste und die zweite Komponente der Hauptteilbahn mit den jeweiligen Seitenabschnitten werden dann übereinander gefördert und miteinander verbunden (Anspruch 4).

Es sei an dieser Stelle auch ausdrücklich darauf hingewiesen, dass die Erfindung auch solche Inkontinenzwegwerfwindeln erfasst, bei denen jeweils nur die hinteren oder vorderen Seitenabschnitte konturiert ausgebildet sind, also die jeweiligen anderen Seitenabschnitte in einem eingangs erwähnten nicht kontinuierlichen Cut-and-place-Verfahren mit insbesondere rechteckförmigen Materialabschnitten angefügt werden (Ansprüche 5 bzw. 6).

Nach einer weiteren Ausführungsform des erfindungsgemäßen Verfahrens von besonderer Bedeutung erweist es sich als vorteilhaft, wenn in der jeweiligen Seitenabschnittbahn zusätzlich zu dem erwähnten Abtrennvorgang ein zum jeweiligen inneren Rand der jeweiligen Seitenabschnittbahn führender Freischnitt ausgeführt wird (Anspruch 7). Durch die Anbringung eines solchen Freischnitts braucht der eigentliche Abtrennvorgang, also der eigentliche Formschnitt zur Konturierung der Beinausschnitte, in Querrichtung nicht die gesamte Breite der betreffenden Seitenabschnittbahn zu überfangen; er braucht also insbesondere nicht den gesamten Fügebereich von Seitenabschnittbahn und Hauptteilbahn zu erfassen. Dennoch ist durch den zum Rand hin geführten Freischnitt sichergestellt, dass zum Entfalten im Anschluss an den Abtrennvorgang die vorderen und hinteren Seitenabschnitte wirklich vereinzelt sind und sich widerstandslos voneinander beabstanden lassen.

Es erweist sich in prozesstechnischer Hinsicht als besonders vorteilhaft, wenn der erwähnte Freischnitt in der endlosen Seitenabschnittbahn vor dem Anfügen an die Hauptteilbahn gebildet wird (Anspruch 8).

In weiterer Ausbildung der Erfindung erweist es sich als vorteilhaft, wenn der Freischnitt eine sich vom inneren Rand der Seitenabschnittbahn erweiternde, insbesondere im wesentlichen Y- oder V-förmige Gestalt oder eine Gestalt nach Art einer geschweiften Klammer aufweist (Anspruch 9). Mit dieser Verfahrensvariante wird nämlich auf technisch besonders elegante Weise sichergestellt, dass im Zuge der Ausführung des Abtrennvorgangs die jeweilige Seitenabschnittbahn über ihre gesamte Länge getrennt, also abgelängt, wird, und zwar auch im Bereich der Einfaltung der Hauptteilbahn, wo möglicherweise ein erforderlicher Gegendruck der Schneidkomponenten nicht immer in hinreichender Weise bereitgestellt werden kann.

Nach einer weiteren Verfahrensvariante ist die Hauptteilbahn im Zuge des Einfaltens, des Fügens mit den Seitenabschnittbahnen und des Ausführens des Abtrennvorgangs über einen einzigen Drehkörper geführt (Anspruch 10).

Beim Einfalten der endlos zugeführten Hauptteilbahn ist es erforderlich, dass die Hauptteilbahn mit einer entsprechend zu berechnenden Geschwindigkeitskurve, d. h. mit einer zyklisch variierenden Geschwindigkeit, zugeführt wird. Um dies zu realisieren, erweist es sich als vorteilhaft, wenn die Geschwindigkeitskurve unter Verwendung einer dem Einfaltvorgang vorgeordneten und entsprechend dem Entfaltvorgang nachgeordneten Tänzeranordnung ausgeführt wird (Anspruch 11). Die Tänzeranordnung bildet einen Speicher für die zum Einfalten zusätzlich benötigte Bahnlänge bzw. für die beim Entfalten zusätzlich abzuführende Bahnlänge.

Gegenstand der Erfindung ist auch eine Inkontinenzwegwerfwindelherstellungsvorrichtung mit den Merkmalen der Ansprüche 12 bis 14.

Nachfolgend werden vorteilhafte Bemessungsangaben der Inkontinenzwegwerfwindel angegeben:

Die Länge der Seitenabschnitte, also deren Erstreckung in Windellängsrichtung beträgt vorzugsweise mindestens 15 cm, insbesondere mindestens 20 cm, weiter insbesondere mindestens 25 cm. Es erweist sich weiterhin als vorteilhaft, wenn die Länge der Seitenabschnitte mindestens 10 %, insbesondere mindestens 15 %, weiter insbesondere mindestens 20 % und weiter insbesondere mindestens 22 %, insbesondere jedoch höchstens 40 % und weiter insbesondere höchstens 35 % der Gesamtlänge der Inkontinenzwegwerfwindel beträgt. Vorteilhafterweise beträgt die Gesamtlänge der Inkontinenzwegwerfwindel 50-120 cm, insbesondere 60-110 cm und weiter insbesondere 70-110 cm. Weiterhin erweist es sich als vorteilhaft, wenn die vorderen Seitenabschnitte eine geringere, insbesondere eine um mindestens 5 %, weiter insbesondere um mindestens 10 %, weiter insbesondere um mindestens 15 % und weiter insbesondere um höchstens 50 % geringere Längserstreckung aufweisen als die hinteren Seitenabschnitte. In Weiterbildung der Erfindung erweist es sich als vorteilhaft, wenn die Breite der Seitenabschnitte, also die Erstreckung der Seitenabschnitte über den Seitenrand des Windelhauptteils hinaus 10-45 cm, insbesondere 13-35 cm, weiter insbesondere 15-27 cm beträgt. Vorzugsweise weisen die vorderen Seitenabschnitte die gleiche Breite auf wie die hinteren Seitenabschnitte. In Weiterbildung der Erfindung erweist es sich als vorteilhaft, wenn die hinteren Seitenabschnitte eine größere, vorzugsweise eine um mindestens 10 %, insbesondere eine um mindestens 15 % größere Flächenerstreckung (gemessen in cm²?) aufweisen als die vorderen Seitenabschnitte.

Es erweist sich weiter als vorteilhaft, wenn die vorderen und/oder hinteren Seitenabschnitte aus einem Vliesstoffmaterial gebildet sind, oder ein Vliesstoffmaterial umfassen, da sich derartige beispielsweise gegenüber Folien eher voluminöse, loftige dreidimensionale Vliesstoffmaterialien eignen, in der erfindungsgemäßen Weise aus dem Prozess als Schnittabfall abgeführt zu werden.

Die Vliesstoffe können Fasern aus PE, PP, PET, Rayon, Cellulose, PA und Mischungen dieser Fasern enthalten. Auch Bi- oder Multikomponentenfasern sind denkbar und vorteilhaft. Vorteilhaft sind insbesondere Kardenvliese, Spinnvliese, wasserstrahlvernadelte Vliese, SM-Vliese, SMS-Vliese, SMMS-Vliese oder auch Laminate aus einer oder mehreren dieser Vliesarten, wobei S für Spunbond- und M für Meltblown-Vliesschichten steht.

Die Hauptteilbahn umfasst vorzugsweise ein Vliesstoffmaterial und/oder ein Saugkörpermaterial und/oder ein Backsheetmaterial. Bei dem Backsheetmaterial kann es sich insbesondere um ein Folienmaterial oder ein flüssigkeitsundurchlässiges Vliesmaterial oder ein Vlies/Folien-Laminat handeln.

Weitere Merkmale, Einzelheiten und Vorteile des erfindungsgemäßen Verfahrens und der erfindungsgemäßen Vorrichtung ergeben sich aus den beigefügten Patentansprüchen und aus der zeichnerischen Darstellung und nachfolgenden Beschreibung bevorzugter Ausführungsformen der Erfindung. In der Zeichnung zeigt:
- Figur 1: eine schematische Draufsicht auf eine herzustellende Inkontinenzwegwerfwindel;
- Figur 2: eine schematische Darstellung der Zuführ-, Einfalt-, Füge-, Abtrenn- und Entfaltvorgänge nach dem erfindungsgemäßen Verfahren;
- Figur 3: eine schematische Ansicht einer erfindungsgemäßen Vorrichtung zur Durchführung des erfindungsgemäßen Herstellungsverfahrens;
- Figur 4: eine perspektivische Ansicht der Vorrichtung nach Figur 3;
- Figur 5: eine schematische Darstellung der Herstellungsvorgänge nach einer weiteren Ausführungsform des erfindungsgemäßen Verfahrens;
- Figur 6: den Verbund aus Hauptteilbahn und konturierten Seitenabschnitten nach dem Verfahren gemäß Figur 5;
- Figuren 7 und 8: Ansichten einer Vorrichtung zur Durchführung des Verfahrens gemäß Figuren 7 und 8;
- Figur 9: eine schematische Darstellung der Verfahrensvorgänge nach einer weiteren Ausführungsform des erfindungsgemäßen Verfahrens, im Zuge deren nur die hinteren Seitenabschnitte konturiert werden, und
- Figur 10: den Verbund aus Hauptteilbahn mit hinteren und vorderen Seitenabschnitten gemäß Verfahren nach Figur 9;
- Figur 11: Draufsicht auf eine vereinzelte Inkontinenzwegwerfwindel erhalten nach dem Verfahren gemäß Figuren 9, 10.

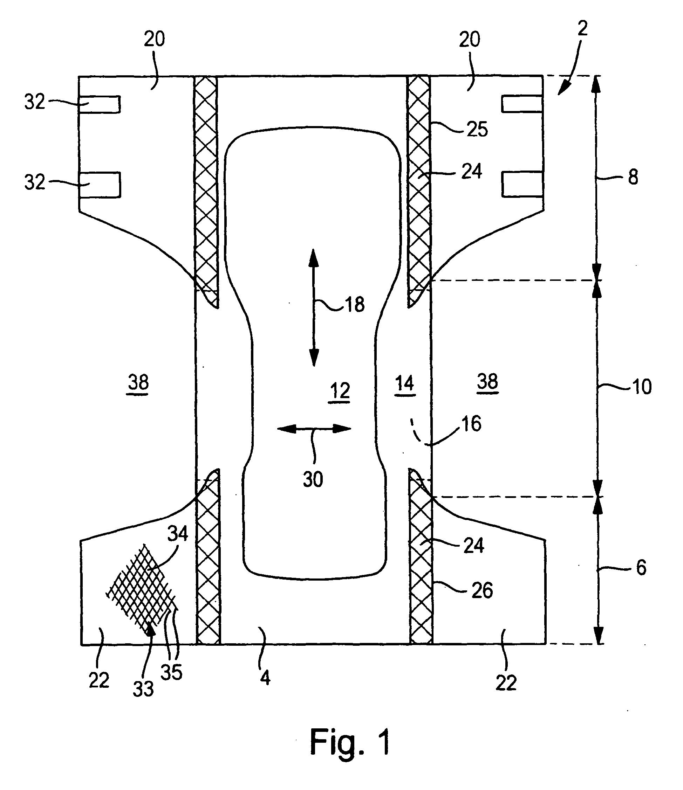

Figur 1 zeigt schematisch eine Draufsicht auf eine Innenseite, also eine körperzugewandte Seite einer öffen- und schließbaren absorbierenden Inkontinenzwegwerfwindel 2 in eben ausgefaltetem Zustand. Die Inkontinenzwegwerfwindel 2 umfasst einen Hauptteil 4 mit einem Vorderbereich 6, einem Rückenbereich 8 und einem in Längsrichtung dazwischen liegenden Schrittbereich 10. Außerdem angedeutet ist ein Saugkörper 12, der üblicherweise zwischen chassisbildenden Materialien des Hauptteils 4, also insbesondere zwischen einem flüssigkeitsdurchlässigen aus einem Vliesstoffmaterial gebildeten Topsheet 14 und einem im Wesentlichen flüssigkeitsundurchlässigen aus einem Folienmaterial gebildeten Backsheet 16 des Hauptteils 4 angeordnet ist. Das Backsheet 16 kann auch aus einem flüssigkeitsundurchlässigen Vliesstoff oder einem Vlies/Folien-Laminat gebildet sein, wobei die Vlieslage dann außen und die Folienlage innen zum Saugkörper gerichtet zu liegen kommt. Dies vermittelt der Inkontinenzwegwerfwindel 2 eine textile Anmutung. Seitlich neben Längsrändern des Saugkörpers 12 sind nicht dargestellte elastische Elemente an den Hauptteil 4, insbesondere zwischen Topsheet 14 und Backsheet 16 angefügt. Die elastischen Elemente verlaufen im Wesentlichen in Längsrichtung 18 der Inkontinenzwegwerfwindel, also mit einer wesentlichen Komponente in Längsrichtung 18, wobei sie einen gekrümmten Verlauf entlang des dem Schrittbereich 10 zuzuordnenden Abschnitts der Beinöffnungen nehmen können.

Die Inkontinenzwegwerfwindel 2 umfasst des Weiteren hintere Seitenabschnitte 20 und vordere Seitenabschnitte 22, die als Vliesstoffkomponenten beidseits an den Hauptteil 4 angefügt sind. Die Seitenabschnitte 20, 22 sind vorzugsweise mittels Ultraschallschweißung an den Hauptteil 4 angefügt. Die Seitenabschnitte 20, 22 sind in einem schraffiert dargestellten Seitenrandbereich 24 mit chassisbildenden Materialien des Hauptteils 4, also beispielsweise mit dem Backsheet 16 und/oder dem Topsheet 14 unlösbar verbunden. Die Seitenabschnitte 20, 22 erstrecken sich über vordere und hintere Längsränder 25, 26 des Hauptteils 4 in einer Querrichtung 30 hinaus. Die hinteren seitlichen Längsränder 25, 26 des Hauptteils 4 begrenzen diejenigen Längsrandbereiche des Hauptteils, an die die Seitenabschnitte 20, 22 angefügt sind und über welche die Seitenrandabschnitte 20, 22 sich in Querrichtung hinaus erstrecken. Die Längserstreckung der vorderen und hinteren Längsränder 25, 26 des Hauptteils 4 definiert damit auch die Längserstreckung des Vorderbereichs 6 und des Rückenbereichs 8 des Hauptteils 4 und auch der Inkontinenzwegwerfwindel, wie dies Figur 1 veranschaulicht. Die Seitenabschnitte 20, 22 sind dafür gedacht und bestimmt, im angelegten Zustand der Inkontinenzwegwerfwindel 2 miteinander verbunden zu werden, um einen in Umfangsrichtung durchgehenden Hüftbereich des Hygieneartikels zu bilden. Dabei werden jeweils die auf einer Seite des Hauptteils 4 vorgesehenen Seitenabschnitte 20, 22 miteinander verbunden. Hierzu sind an den hinteren Seitenabschnitten 20 mechanische Verschlussmittel 32, insbesondere mit mechanischen Verschlusshilfen wie Kletthaken, vorgesehen, welche auf der Außenseite der vorderen Seitenabschnitte 20, 22 lösbar festlegbar sind. Vorzugsweise sind die Verschlussmittel 32 außerdem auf der Außenseite des Hauptteils 4 lösbar festlegbar. Sowohl die vorderen Seitenabschnitte 22 als auch die hinteren Seitenabschnitte 20 sind aus einem Vliesstoffmaterial, im beispielhaft dargestellten Fall aus einem PP-Spinnvlies, gebildet. Das Flächengewicht des Vliesstoffmaterials der vorderen Seitenabschnitte 22 beträgt 30 g/m². Die Faserstärke der das Vliesstoffmaterial bildenden Fasern beträgt 2 dtex. Außen- und Innenseite des Spinnvliesstoffs weisen ein in Figur 1 nur angedeutetes Prägemuster 33 auf. Die durch Heißkalanderprägung erzeugten Fügebereiche des Prägemusters 33 sind durch eine Vielzahl von Prägelinien 35 gebildet, nämlich durch zwei Gruppen von innerhalb einer Gruppe jeweils parallel verlaufenden Prägelinien 35, wobei sich die Prägelinien der einen Gruppe mit den Prägelinien der anderen Gruppe zur Ausbildung eines regelmäßigen Rautenmusters unter einem Winkel von beispielhaft 33 Grad schneiden, so dass inselartig angeordnete rautenförmige Schlaufenbereiche 34 von Prägelinien 35 umgeben sind. Die Prägelinien 35 haben im dargestellten Fall eine Breite von 1,0 mm und eine Prägetiefe von 0,6 mm. Der Abstand zweier benachbarter parallel verlaufender Prägelinien 35 beider Gruppen von Linien beträgt 4,7 mm. Die Prägefläche, also die Summe der Fläche aller Prägelinien 35 bezogen auf die Gesamtfläche des Prägemusters (Prägelinien 35 + Schlaufenbereiche 34), beträgt 32 %. Die Verschlussmittel 32 der hinteren Seitenabschnitte 20 sind mit diesen Schlaufenbereichen 34 sicher in Eingriff bringbar. Die Über-Bauch-Haltekräfte zwischen den Verschlussmitteln 32 und der Außenseite der vorderen Seitenabschnitte 22 betragen vorzugsweise mindestens 58 N/25mm.

Das Flächengewicht des Vliesstoffmaterials der hinteren Seitenabschnitte 20 beträgt im dargestellten Fall 25 g/m². Ein Schlaufenbereiche und Fügebereiche bildendes Prägemuster ist nicht vorgesehen. Die Über-Bauch-Haltekräfte zwischen den Verschlussmitteln 32 und der Außenseite der hinteren Seitenabschnitte 20 sind daher geringer als die Über-Bauch-Haltekräfte zwischen den Verschlussmitteln 32 und der Außenseite der vorderen Seitenabschnitte 22, sie betragen vorzugsweise aber gleichwohl mindestens 15 N/25mm gemessen nach der in EP 1915977 A1 beschriebenen Prüfmethode. Wie aus Figur 1 erkennbar ist, weisen die hinteren Seitenabschnitte 20 zudem eine größere Flächenerstreckung auf als die vorderen Seitenabschnitte 22.

Die vorderen und hinteren Seitenabschnitte 20, 22 unterscheiden sich damit in zumindest drei ihrer Primäreigenschaften, nämlich dem Flächengewicht, der Verschlusskraft und der Flächenerstreckung. Der Unterschied in der Verschlusskraft zwischen den vorderen und hinteren Seitenabschnitten veranlasst den Benutzer, die Verschlussmittel 32 bevorzugt an den vorderen Seitenabschnitten 22 festzulegen, was der Passform der Windel förderlich ist. Wie aus Figur 1 weiter zu erkennen ist, sind Beinöffnungsbereiche 38 durch zum Schrittbereich 10 hin kurvenförmig konturierte vordere und hintere Seitenabschnitte 20, 22 gebildet. Zusätzlich wäre auch eine sanduhrförmige Konturierung des Hauptteils 4 denkbar. Unter einer sanduhrförmigen Konturierung des Hauptteils 4 wird hier jede Form der Verengung des Hauptteils 4 im Schrittbereich 10 verstanden, bei der der Schrittbereich 10 des Hauptteils 4 eine geringere Erstreckung in Querrichtung 30 aufweist als Vorderbereich 6 und/oder Rückenbereich 8 des Hauptteils.

Nachfolgend wird das erfindungsgemäße Verfahren zur Herstellung der vorstehend beschriebenen Inkontinenzwegwerfwindel näher erläutert:

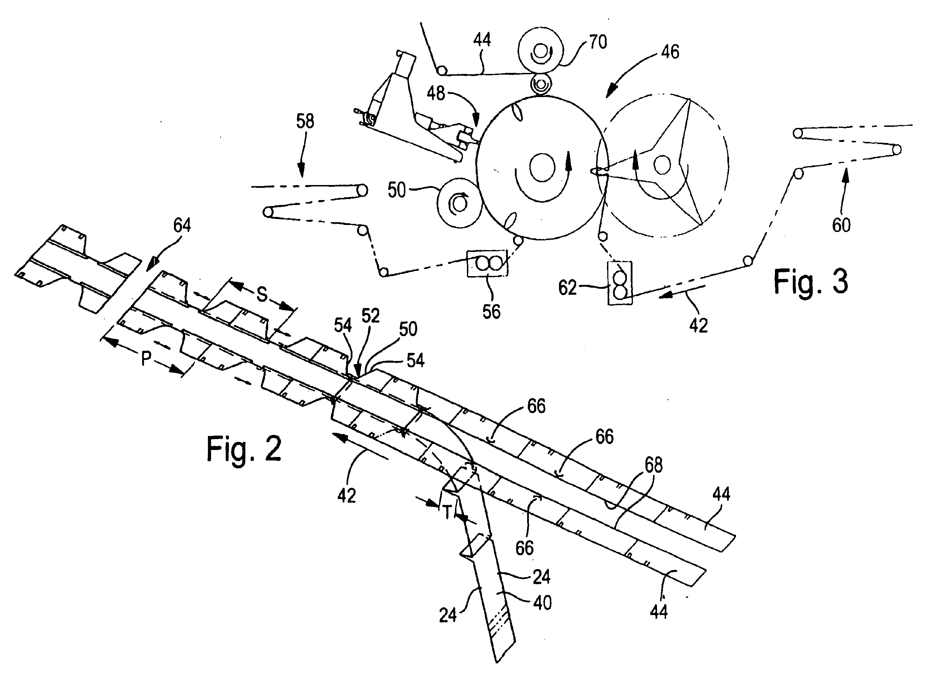

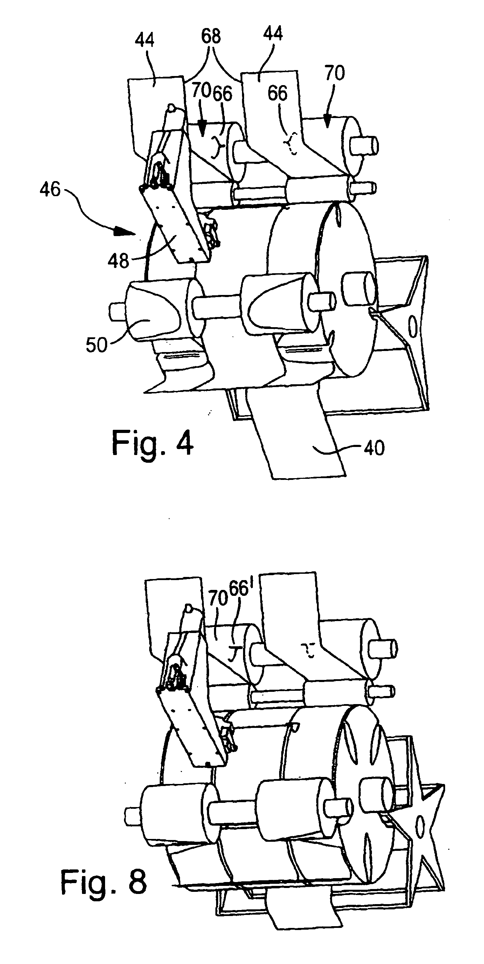

Figur 2 zeigt schematisch die verschiedenen hier im Mittelpunkt des Interesses stehenden Schritte bei der Herstellung der erfindungsgemäßen Inkontinenzwegwerfwindel 2. Es wird eine Hauptteilbahn 40 endlos in Maschinenrichtung 25 zugeführt ebenso wie zwei Seitenabschnittbahnen 44, welche die späteren beidseitigen hinteren und vorderen Seitenabschnitte 20, 22 bilden. Bevor die Seitenabschnittbahnen 44 an Seitenrandbereiche 24 der Hauptteilbahn 40 appliziert und angefügt werden, wird die Hauptteilbahn 40 quer zur Maschinenrichtung 42 und quer zur Bahnebene eingefaltet, was auch anhand der in Figuren 3 und 4 dargestellten Vorrichtung 46 ersichtlich ist. Die Einfalttiefe T ist dabei derart gewählt, dass im Wesentlichen die gesamte Länge des späteren Schrittbereichs 10 aus der Bahnebene weggefaltet ist. Die Einfalttiefe T beträgt dabei 0,5 x (Produktlänge P - Seitenabschnittlänge S). Die Seitenabschnittbahnen 44 werden dann beidseits an die Hauptteilbahn 40 appliziert und durch Vorbeiführen an einer Ultraschallsonotrode 48 unlösbar mit der Hauptteilbahn 40 verbunden, und zwar in Maschinenrichtung 42 im Wesentlichen durchgehend (auch wenn einzelne Schweißpunkte hierfür Verwendung finden). Es folgt dann die Ausführung eines Abtrennvorgangs, wobei mittels einer Schneidwalze 50 ein Formschnitt 52 in den Seitenabschnittbahnen 44 ausgeführt wird, wodurch zum einen Ränder 54 der Seitenabschnitte 20, 22 konturiert und zum anderen die Seitenabschnitte 20, 22 im Bereich des Formschnitts 52 abgetrennt werden.

Sodann wird der Verbund aus Hauptteilbahn 40 und Seitenabschnittbahnen 44 vermittels einer Abzugseinrichtung 56 beschleunigt und dabei entfaltet. Dabei wird der Schrittbereich 10 wieder in die Bahnebene gebracht und hinterer und vorderer Seitenabschnitt 20, 22 werden voneinander beabstandet. Zur Aufnahme dieser eingefalteten Länge ist eine Tänzeranordnung 58 der Abzugseinrichtung 56 nachgeordnet. Eine entsprechende Tänzereinrichtung 60 und Zuführeinrichtung 62 ist dem vorstehend beschriebenen Prozess vorgeordnet.

Weiter zeigt Figur 2 andeutungsweise den Schritt 64 der Vereinzelung der Inkontinenzwegwerfwindel 2.

Durch den erfindungsgemäßen Vorgang des Einfaltens der Hauptteilbahn 4 lässt sich der Schnittabfall bei den Seitenabschnittbahnen 44 im Zuge des Konturierens der einander zugewandten Ränder 54 der hinteren und vorderen Seitenabschnitte 20, 22 auf das minimale Maß reduzieren. Dennoch kann mit endlosen Seitenabschnittbahnen 44 gearbeitet werden, was sich prozesstechnisch als vorteilhaft erweist.

Auf ein weiteres besonders vorteilhaftes Verfahrensdetail sei weiter hingewiesen: Noch in den endlosen Seitenabschnittbahnen 44 und vorzugsweise vor dem Applizieren an Seitenrandbereichen 24 der Hauptteilbahn 4 wird ein beispielhaft in Form einer geschweiften Klammer ausgebildeter Freischnitt 66 vorgesehen, der sich bis zu den einander zugewandten inneren Längsrändern 68 der Seitenabschnittbahnen 44 erstreckt. Auf diese Weise braucht der Abtrennvorgang in Form des Formschnitts 52 nicht bis zu den Längsrändern 68 und damit in den Überlappungsbereich mit der Hauptteilbahn 40 erstreckt zu werden, sondern er braucht nur diesen Freischnitt 66 zu erreichen, um eine sichere Abtrennung von hinteren und vorderen Seitenabschnitten 20, 22 und damit eine Entfaltbarkeit des Verbunds aus Hauptteilbahn 40 und Seitenabschnittbahnen 44 sicherzustellen. Dieser Freischnitt 66 ist im beispielhaften und bevorzugten Fall derart ausgebildet, dass er sich von den Längsrändern 68 weg erweitert. Er könnte auch V-förmig oder Y-förmig oder sich in anderer Form erweiternd ausgebildet werden, um den erfindungsgemäßen Vorteil zu gewährleisten. Grundsätzlich könnte auch eine zum Längsrand 68 hin offene Schlitzung der Seitenabschnittbahnen 44 für den intendierten Zweck ausreichen. Es hat sich aber gezeigt, dass durch die sich erweiternde Ausbildung des Freischnitts 66 auch dann eine prozesstechnisch stabile und sichere Abtrennung des hinteren und vorderen Seitenabschnitts 20, 22 realisiert werden kann, wenn im Bereich der Einfaltung der Hauptteilbahn (im Scheitel der Formschnitts 52) der Gegendruck für die Schneidwalze 50 möglicherweise nicht hinreichend sein sollte. Der erwähnte Freischnitt 66 wird im beispielhaft dargestellten Fall von einer Freischneideinrichtung 70 ausgeführt. Es könnte als Freischnitt auch ein insbesondere rundes oder ovales Flächenstück ausgeschnitten oder ausgestanzt werden.

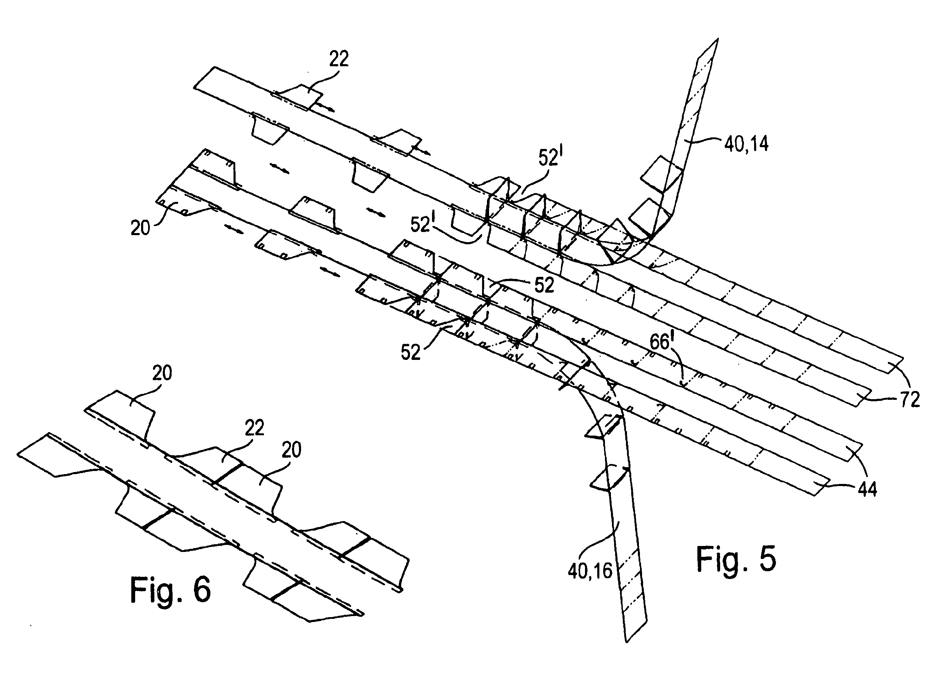

Figuren 5 und 6 zeigen eine weitere Ausführungsform des erfindungsgemäßen Verfahrens, bei dem die hinteren und vorderen Seitenabschnitte 20, 22 von unterschiedlichen Seitenabschnittbahnen, nämlich die hinteren Seitenabschnitte 20 von beidseitigen ersten Seitenabschnittbahnen 44 und die vorderen Seitenabschnitte 22 von beidseitigen zweiten Seitenabschnittbahnen 72 gebildet werden. Die Hauptteilbahn ist wiederum mit Bezugszeichen 40 bezeichnet; sie umfasst aber nur eine Komponente in Form des Backsheets 16 als endlose Backsheetbahn. In einem ersten Applikationsschritt wird diese Komponente der Hauptteilbahn 40 endlos zugeführt und mit einer gegenüber dem ersten Ausführungsbeispiel größeren Einfalttiefe eingefaltet, so dass ihre in der Bahnebene verbleibende Länge der Länge der hinteren Seitenabschnitte 20 (je Produkt) entspricht. Die ersten Seitenabschnittbahnen 44 werden dann wie aus Figur 5 ersichtlich zugeführt und entsprechend dem ersten Ausführungsbeispiel an Seitenrandbereichen 24 unlösbar fixiert. Sodann wird die Konturierung, d. h. der Abtrennvorgang in Form eines entsprechenden Formschnitts 52 in den Seitenabschnittbahnen 44 ausgeführt und der Verbund aus Hauptteilbahn 40 und Seitenabschnittbahnen 44 entfaltet.

Insbesondere parallel hierzu wird - wie in Figur 5 beispielhaft oben abgebildet ist - eine weitere Komponente der Hauptteilbahn 40 in Form des Topsheets 14 als endlose Topsheetbahn zugeführt und in entsprechender Weise eingefaltet, so dass in diesem Fall die in der Bahnebene verbleibende Länge je Produkt der Länge der vorderen Seitenabschnitte 22 entspricht. Sodann werden beidseits die zweiten Seitenabschnittbahnen 72 zugeführt und unlösbar appliziert, woraufhin ein weiterer Formschnitt 52' ausgeführt wird. Danach erfolgt eine Entfaltung. Die beiden Bahnen werden dann übereinander geführt und miteinander verbunden, wobei zwischen Topsheetbahn und Backsheetbahn noch ein Saugkörper (nicht dargestellt) angeordnet werden kann. Die Bahn weist dann die in Figur 6 dargestellte Gestalt auf.

Im Unterschied zum ersten Ausführungsbeispiel sind die jeweiligen vorderen Seitenabschnitte 22 von den unmittelbar angrenzenden hinteren Seitenabschnitten 20 des benachbarten Produkts bereits separiert, noch bevor die einzelnen Produkte vereinzelt werden.

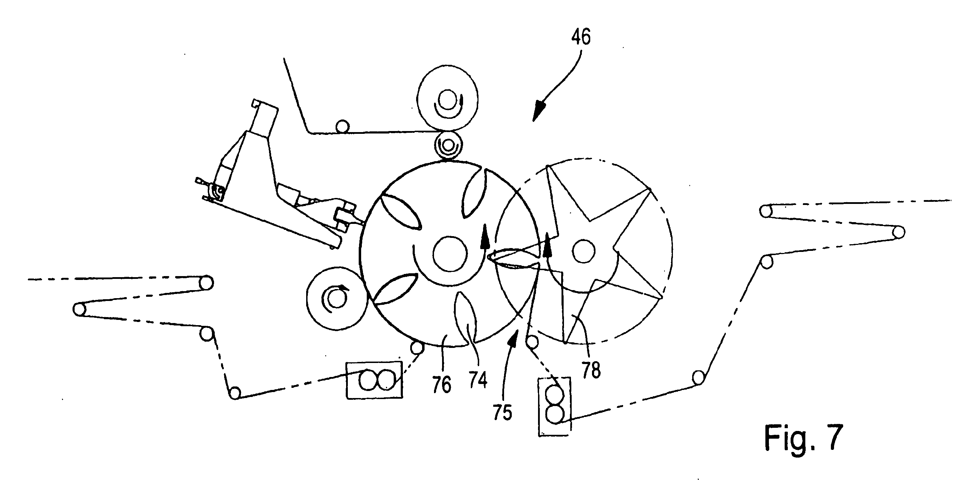

Die Figuren 7 und 8 zeigen eine den Figuren 3 und 4 grundsätzlich entsprechende Vorrichtung 46 zur Durchführung des Verfahrens, wobei Einfaltöffnungen 74 bei einer Einfalteinrichtung 75 mit einem ersten Drehkörper 76 und einem drehenden Verdrängerorgan 78 der Vorrichtung 46 für eine entsprechend tiefere Einfaltung der Hauptteilbahn 40 ausgebildet ist. Ferner ist die Freischnitteinrichtung 70 mit einer aus Figur 8 ersichtlichen Messerform zur Ausbildung eines geeigneten Freischnitts 66' wiederum mit sich jedoch nur einseitig erweiternder Gestalt ausgebildet.

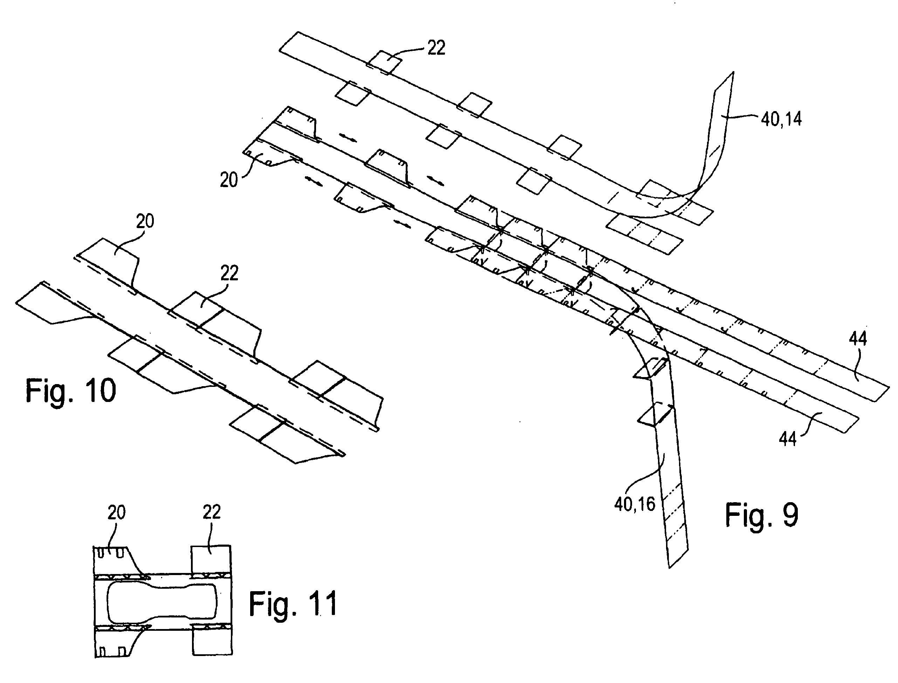

Die Figuren 9 bis 11 verdeutlichen eine weitere Variante des erfindungsgemäßen Verfahrens, wobei nur die jeweiligen hinteren Seitenabschnitte 20 durch Zuführen und Applizieren endloser Seitenabschnittbahnen 44 an eine zuvor gefaltete Komponente (z.B. Backsheetbahn) der Hauptteilbahn 40 gebildet sind. Die jeweiligen vorderen Seitenabschnitte 22 sind im Cut-and-place-Verfahren getaktet auf eine andere Komponente (z.B. Topsheetbahn) der Hauptteilbahn 40 aufgebracht und dort unlösbar fixiert.

## Patentansprüche

1. Verfahren zum Herstellen einer absorbierenden Inkontinenzwegwerfwindel (2), mit einem einen Saugkörper (12) aufweisenden Hauptteil (4), umfassend einen Vorderbereich (6) mit vorderen seitlichen Längsrändern (26), einen Rückenbereich (8) mit hinteren seitlichen Längsrändern (25) und einen dazwischen angeordneten, zwischen den Beinen eines Benutzers zu liegen kommenden Schrittbereich (10), und mit beidseits an den Rückenbereich (8) angefügten hinteren Seitenabschnitten (20) und beidseits an der Vorderbereich (6) angefügten vorderen Seitenabschnitten (22), welche sich in einer Querrichtung (30) der Inkontinenzwegwerfwindel (2) über die seitlichen vorderen bzw. hinteren Längsränder (25, 26) des Hauptteils (4) hinaus erstrecken und in einer Längsrichtung (18) der Inkontinenzwegwerfwindel (2) voneinander beabstandet sind, wobei die hinteren und vorderen Seitenabschnitte (20, 22) zum Anlegen der Inkontinenzwegwerfwindel (2) miteinander lösbar verbindbar sind, **dadurch gekennzeichnet, dass** zur Konturierung von beidseitigen Beinöffnungsbereichen (38) der Inkontinenzwegwerfwindel (2) die hinteren Seitenabschnitte (20) und/oder die vorderen Seitenabschnitte (22) durch einen Abtrennvorgang von endlos zugeführten Seitenabschnittbahnen (44, 72) gebildet und dabei konturiert werden, und dass eine endlos zugeführte Hauptteilbahn (40) vor dem Anfügen der endlos zugeführten Seitenabschnittbahnen (44, 72) quer zur Bahnebene und quer zur Maschinenrichtung (42) eingefaltet wird, dass dann die Seitenabschnittbahnen (44, 72) an die Hauptteilbahn (40) angefügt werden, dass der die Seitenabschnittbahnen erfassende Abtrennvorgang (52) beidseits ausgeführt wird, so dass dann der Verbund aus Hauptteilbahn (40) und angefügten Seitenabschnittbahnen (44, 72) in Maschinenrichtung (42) beschleunigt und dabei entfaltet werden kann, wobei die vorderen und hinteren Seitenabschnitte (20, 22) in der Längsrichtung (18) voneinander beabstandet werden können.

2. Verfahren nach Anspruch 1, **dadurch gekennzeichnet, dass** die Tiefe (T) der Einfaltung der Hauptteilbahn (40) derart ist, dass die in der Bahnebene zum Anfügen der Seitenabschnittbahnen (44) zur Verfügung stehende Länge der Hauptteilbahn (40) je Inkontinenzwegwerfwindel der Länge der Seitenabschnitte (20, 22) je Inkontinenzwegwerfwindel entspricht.

3. Verfahren nach Anspruch 1, **dadurch gekennzeichnet, dass** die jeweiligen vorderen und hinteren Seitenabschnitte (20, 22) von verschiedenen endlos zugeführten Seitenabschnittbahnen (44, 72) gebildet werden, indem in einem ersten Applikationsschritt beidseits eine erste Seitenabschnittbahn (44) an die zuvor eingefaltete Hauptteilbahn (40) angefügt und daran anschließend ein erster die ersten Seitenabschnittbahnen (44) erfassender Abtrennvorgang (52) beidseits ausgeführt wird und die Hauptteilbahn (40) wieder entfaltet wird, und dass dann in einem zweiten Applikationsschritt beidseits eine zweite Seitenabschnittsbahn (72) an die zuvor erneut eingefaltete Hauptteilbahn (40) angefügt und daran anschließend ein zweiter die zweiten Seitenabschnittbahnen (72) erfassender Abtrennvorgang (52) beidseits ausgeführt wird.

4. Verfahren nach Anspruch 1, **dadurch gekennzeichnet, dass** die jeweiligen vorderen und hinteren Seitenabschnitte (20, 22) von verschiedenen endlos zugeführten Seitenabschnittbahnen (44, 72) gebildet werden, dass dabei in einem ersten Applikationsschritt beidseits eine erste Seitenabschnittbahn (44) an eine zuvor eingefaltete erste Komponente der Hauptteilbahn (40) angefügt und daran anschließend ein erster die ersten Seitenabschnittbahnen (44) erfassender Abtrennvorgang (52) beidseits ausgeführt wird und die Hauptteilbahn (40) wieder entfaltet wird, und dass in einem zweiten, insbesondere parallel zu dem ersten ablaufenden Applikationsschritt beidseits eine zweite Seitenabschnittsbahn (72) an eine zuvor eingefaltete zweite Komponente der Hauptteilbahn (40) angefügt und daran anschließend ein zweiter die zweiten Seitenabschnittbahnen (72) erfassender Abtrennvorgang (52) beidseits ausgeführt wird und die Hauptteilbahn (40) wieder entfaltet wird, dass die erste und die zweite Komponente der Hauptteilbahn (40) mit den jeweiligen Seitenabschnitten übereinander gefördert und miteinander verbunden werden.

5. Verfahren nach Anspruch 1, **dadurch gekennzeichnet, dass** die jeweiligen vorderen und hinteren Seitenabschnitte (20, 22) von verschiedenen Seitenabschnittbahnen gebildet werden, indem in einem ersten Applikationsschritt beidseits hintere oder vordere Seitenabschnitte (20, 22) in einem cut-and place-Verfahren an die Hauptteilbahn (40) angefügt werden, und dass dann in einem zweiten Applikationsschritt beidseits eine endlos zugeführte Seitenabschnittsbahn (44) an die zuvor eingefaltete Hauptteilbahn (40) angefügt und daran anschließend ein die Seitenabschnittbahnen (44) erfassender Abtrennvorgang beidseits ausgeführt wird.

6. Verfahren nach Anspruch 5, **dadurch gekennzeichnet, dass** in dem ersten Applikationsschritt hintere oder vordere Seitenabschnitte (20, 22) in einem cut-and place-Verfahren an eine erste Komponente der Hauptteilbahn (40) angefügt werden und die Hauptteilbahn (40) wieder entfaltet wird, und dass in einem zweiten Applikationsschritt beidseits eine endlos zugeführte Seitenabschnittsbahn (44) an eine zuvor eingefaltete zweite Komponente der Hauptteilbahn (40) angefügt und daran anschließend ein die Seitenabschnittbahnen (44) erfassender Abtrennvorgang beidseits ausgeführt wird und die Hauptteilbahn (40) wieder entfaltet wird, und dass die erste und die zweite Komponente der Hauptteilbahn (40) mit den jeweiligen Seitenabschnitten (20, 22) übereinander gefördert und miteinander verbunden werden.

7. Verfahren nach einem der vorstehenden Ansprüche, **dadurch gekennzeichnet, dass** in der jeweiligen Seitenabschnittbahn (44, 72) zusätzlich zu dem erwähnten Abtrennvorgang (52) ein zum jeweiligen inneren Rand (68) der jeweiligen Seitenabschnittbahn (44, 72) führender Freischnitt (66) ausgeführt wird.

8. Verfahren nach Anspruch 7, **dadurch gekennzeichnet, dass** der Freischnitt (66) in der endlosen Seitenabschnittbahn (44, 72) vor dem Anfügen an die Hauptteilbahn (44) gebildet wird.

9. Verfahren nach Anspruch 7 oder 8, **dadurch gekennzeichnet, dass** der Freischnitt (66) eine sich vom inneren Rand (68) der Seitenabschnittbahn (44, 72) erweiternde, insbesondere im wesentlichen Y- oder V-förmige Gestalt oder eine Gestalt nach Art einer geschweiften Klammer aufweist.

10. Verfahren nach einem der vorstehenden Ansprüche, **dadurch gekennzeichnet, dass** die Hauptteilbahn (40) im Zuge des Einfaltens, des Fügens mit den Seitenabschnittbahnen (44, 72) und des Ausführens des Abtrennvorgangs (52) über einen einzigen Drehkörper (76) geführt ist.

11. Verfahren nach einem der vorstehenden Ansprüche, **dadurch gekennzeichnet, dass** eine jeweilige Geschwindigkeitskurve der Hauptteilbahn (40) zur Durchführung des Einfaltens und/oder Entfaltens unter Verwendung einer vorgeordneten und/oder einer nachgeordneten Tänzeranordnung (58, 60) ausgeführt wird.

12. Inkontinenzwegwerfwindelherstellungsvorrichtung (46) zum Herstellen einer Inkontinenzwegwerfwindel nach einem oder mehreren der vorstehenden Ansprüche mit Zuführeinrichtungen (62) für die Hauptteilbahn (40) und für die beidseitigen Seitenabschnittbahnen (44, 72) und mit einer Fügeeinrichtung (48) zum Befestigen der Seitenabschnittbahnen (44, 72) an Längsrandbereichen (24) der Hauptteilbahn (40) und mit einer Schneideinrichtung (50) zur Konturierung der hinteren und/oder vorderen Seitenabschnitte (20, 22), **dadurch gekennzeichnet, dass** in Maschinenrichtung (42) vor der Fügeeinrichtung (48) und vor der Schneideinrichtung (50) eine Einfalteinrichtung (75) für die Hauptteilbahn (40) vorgesehen ist zum Einfalten der Hauptteilbahn (40) quer zur Bahnebene und quer zur Maschinenrichtung (42).

13. Vorrichtung nach Anspruch 12, **dadurch gekennzeichnet, dass** die Einfalteinrichtung (75), die Fügeeinrichtung (48) und die Schneideinrichtung (50) bei demselben Drehkörper (76) ausgebildet bzw. angeordnet sind.

14. Vorrichtung nach Anspruch 12 oder 13, **dadurch gekennzeichnet**, **dadurch gekennzeichnet, dass** der Einfalteinrichtung (75), der Fügeeinrichtung (48) und der Schneideinrichtung (50) eine Tänzeranordnung (60, 58) vorgeordnet und/oder nachgeordnet ist, um die Geschwindigkeitskurve der Hauptteilbahn (40) zum Durchführen des Einfaltens und Entfaltens auszuführen.

## Claims

1. Method for producing an absorbent disposable incontinence diaper (2) having a main part (4) comprising an absorption element (12), the main part having a front region (6) with front longitudinal side edges (26), a rear region (8) having rear longitudinal side edges (25) and a crotch region (10) arranged therebetween and placed between the legs of a user, and the diaper having rear side sections (20) joined to the rear region (8) on both sides and front side sections (22) joined to the front region (6) on both sides, which extend in a transverse direction (30) of the disposable incontinence diaper (2) beyond the respective front and rear longitudinal side edges (25, 26) of the main part (4) and are spaced apart from one another in a longitudinal direction (18) of the disposable incontinence diaper (2), the rear and front side sections (20, 22) being detachably connectable to each other in order to apply the disposable incontinence diaper (2), **characterized in that**, for contouring leg opening regions (38) on both sides of the disposable incontinence diaper (2), the rear side sections (20) and/or the front side sections (22) are formed by a severing operation of side section webs (44, 72) that are endlessly supplied and are contoured in the process, and that an endlessly supplied main part web (40) is folded transversely with respect to the plane of the web and transversely with respect to the machine direction (42) before the endlessly supplied side section webs (44, 72) are joined, that the side section webs (44, 72) are then joined to the main part web (40), that the severing operation (52) covering the side section webs is then carried out on both sides so that the composite comprising the main part web (40) and joined side section webs (44, 72) can then be accelerated in the machine direction (42) and unfolded in the process, wherein the front and rear side sections (20, 22) thereby become spaced apart from each other in the longitudinal direction (18).

2. Method according to claim 1, **characterized in that** the depth (T) of the fold of the main part web (40) is selected such that the length of the main part web (40) available in the web plane for joining the side section webs (44) for each disposable incontinence diaper corresponds to the length of the side sections (20, 22) of each disposable incontinence diaper.

3. Method according to claim 1, **characterized in that** the respective front and rear side sections (20, 22) are formed by different endlessly supplied side section webs (44, 72) by joining a first side section web (44) to the previously folded main part web (40) on both sides in a first application step, followed by performing a first severing operation (52) covering the first side section webs (44) on both sides and unfolding again the main part web (40), and a second side section web (72) is then joined on both sides to the previously again folded main part web (40) in a second application step, followed by a second severing operation (52) covering the second side section webs (72) on both sides.

4. Method according to claim 1, **characterized in that** the respective front and rear side sections (20, 22) are formed by different endlessly supplied side section webs (44, 72), that a first side section web (44) is thereby joined on both sides to a previously folded first component of the main part web (40) in a first application step, and that a first severing operation (52) covering the first side section webs (44) is subsequently performed on both sides and the main part web (40) is unfolded again, and that in a second application step which is performed, in particular, parallel to the first application step, a second side section web (72) is joined on both sides to a previously folded second component of the main part web (40) and a second severing operation (52) covering the second side section webs (72) is subsequently performed on both sides and the main part web (40) is unfolded again, and that the first and the second component of the main part web (40) with the respective side sections are conveyed on top of each other and are connected to each other.

5. Method according to claim 1, **characterized in that** the respective front and rear side sections (20, 22) are formed by different side section webs **in that**, in a first application step, rear or front side sections (20, 22) are joined on both sides to the main part web (40) in a cut-and-place method, and that then in a second application step, an endlessly supplied side section web (44) is joined on both sides to the previously folded main part web (40), followed by a severing operation covering the side section webs (44) on both sides.

6. Method according to claim 5, **characterized in that** in the first application step, rear or front side sections (20, 22) are joined in a cut-and-place-method to a first component of the main part web (40), and the main part web (40) is unfolded again, and that in a second application step an endlessly supplied side section web (44) is joined on both sides to a previously folded second component of the main part web (40) and subsequent thereto, a severing operation covering the side section webs (44) is performed on both sides and the main part web (40) is unfolded again and the first and the second components of the main part web (40) with the respective side sections (20, 22) are conveyed on top of each other and are connected to each other.

7. Method according to any one of the preceding claims, **characterized in that**, in the respective side section web (44, 72), a free cut (66) extending to the respective inner edge (68) of the respective side section web (44, 72) is performed in addition to the mentioned severing operation (52).

8. Method according to claim 7, **characterized in that** the free cut (66) is formed in the endless side section web (44, 72) prior to joining to the main part web (44).

9. Method according to claim 7 or 8, **characterized in that** the free cut (66) has a shape which widens from the inner edge (68) of the side section web (44, 72), in particular, has a substantially Y- or V-shaped form or a shape that corresponds to a curly bracket.

10. Method according to any one of the preceding claims, **characterized in that** the main part web (40) is guided over one single rotary body (76) during folding, joining to the side section webs (44, 72) and performing of the severing operation (52).

11. Method according to any one of the preceding claims, **characterized in that** a respective speed curve of the main part web (40) is performed for performing a folding and/or unfolding action, thereby using one upstream and/or one downstream compensator arrangement (58, 60).

12. Device (46) for producing disposable incontinence diapers for producing a disposable incontinence diaper according to one or more of the preceding claims, comprising supply devices (62) for the main part web (40) and for the side section webs (44, 72) on both sides, and comprising a joining device (48) for mounting the side section webs (44, 72) to longitudinal edge regions (24) of the main part web (40) and comprising a cutting device (50) for providing the rear and/or front side sections (20, 22) with a contour, **characterized in that** a folding device (75) for the main part web (40) is provided in the machine direction (42) upstream of the joining device (48) and upstream of the cutting device (50) for folding the main part web (40) transversely to the web plane and transversely to the machine direction (42).

13. Device according to claim 12, **characterized in that** the folding device (75), the joining device (48) and the cutting device (50) are formed or arranged at the same rotary body (76).

14. Device according to claim 12 or 13, **characterized in that** the folding device (75), the joining device (48) and the cutting device (50) have an upstream and/or downstream compensator arrangement (60, 58) for performing the speed curve of the main part web (40) for performing the folding and unfolding process.

## Revendications

1. Procédé de fabrication d'une couche d'incontinence (2), jetable et absorbante, comprenant une partie principale (4) comportant un corps absorbant (12), laquelle comprend une zone avant (6) avec des bords longitudinaux latéraux avant (26), une zone arrière (8) avec des bords longitudinaux latéraux arrière (25) et une zone d'entrejambe (10) disposée entre elles, venant se placer entre les jambes d'un utilisateur, et comportant des segments latéraux arrière (20) rapportés des deux côtés à la zone arrière (8), et des segments latéraux avant (22) rapportés des deux côtés à la zone avant (6), segments qui s'étendent, dans une direction transversale (30) de la couche d'incontinence jetable (2), sur les bords longitudinaux latéraux avant et arrière (25, 26) de la partie principale (4) et se situent à une certaine distance l'un de l'autre dans une direction longitudinale (18) de la couche d'incontinence jetable (2), les segments latéraux arrière et avant (20, 22) pouvant, pour permettre la mise en place de la couche d'incontinence jetable (2), être assemblés l'un à l'autre d'une manière amovible, **caractérisé en ce que**, pour définir le contour des zones (38) d'ouverture de jambe, des deux côtés, de la couche d'incontinence jetable (2), les segments latéraux arrière (20) et/ou les segments latéraux avant (22) sont formés par une opération de séparation de bandes amenées sans fin (44, 72) de segments latéraux, ce qui en définit le contour, et qu'une bande de partie principale (40), amenée sans fin, est repliée avant assemblage des bandes amenées sans fin de segments latéraux (44, 72), transversalement au plan de la bande et transversalement au sens machine (42), qu'ensuite les bandes de segments latéraux (44, 72) sont assemblées à la bande de partie principale (40), que l'opération de séparation (52), qui reprend les bandes de segments latéraux, est effectuée des deux côtés, de façon qu'il soit alors possible d'accélérer et de fait de déployer l'assemblage de la bande de partie principale (40) et des bandes de segments latéraux assemblés (44, 72) dans le sens machine (42), les segments latéraux avant et arrière (20, 22) pouvant se trouver à une certaine distance l'un de l'autre dans la direction longitudinale (18).

2. Procédé selon la revendication 1, **caractérisé en ce que** la profondeur (T) du déploiement de la bande de partie principale (40) est telle que la longueur de la bande de partie principale (40) de chaque couche d'incontinence jetable à disposition dans le plan de la bande pour l'assemblage des bandes de segments latéraux (44) corresponde à la longueur des segments latéraux (20, 22) de chaque couche d'incontinence jetable.

3. Procédé selon la revendication 1, **caractérisé en ce que** les segments latéraux avant et arrière (20, 22) respectifs sont formés par différentes bandes de segments latéraux (44, 72) amenées sans fin du fait qu'au cours d'une première étape d'application, une première bande de segments latéraux (44) est assemblée des deux côtés à la bande de partie principale (40) repliée puis qu'une première opération de séparation (52) reprenant les premières bandes de segments latéraux (44) est effectuée des deux côtés et que la bande de partie principale (40) est à nouveau déployée, et **en ce qu'**au cours d'une seconde étape d'application, une seconde bande de segments latéraux (72) est assemblée des deux côtés à la bande de partie principale (40) précédemment à nouveau repliée, puis une seconde opération de séparation (52) reprenant les secondes bandes de segments latéraux (72) est effectuée des deux côtés.

4. Procédé selon la revendication 1, **caractérisé en ce que** les segments latéraux avant et arrière (20, 22) respectifs sont formés par différentes bandes de segments latéraux (44, 72) amenées sans fin , **en ce qu'**au cours d'une première étape d'application, une première bande de segments latéraux (44) est assemblée des deux côtés à un premier élément précédemment replié de la bande de partie principale (40) puis une première opération de séparation (52) reprenant les premières bandes de segments latéraux (44) est effectuée des deux côtés et la bande de partie principale (40) est à nouveau déployée, et **en ce qu'**au cours d'une seconde étape d'application se déroulant en particulier parallèlement à la première étape d'application, une seconde bande de segments latéraux (72) est assemblée des deux côtés à un second élément précédemment replié de la bande de partie principale (40) puis une seconde opération de séparation (52) reprenant les secondes bandes de segments latéraux (72) est effectuée des deux côtés et la bande de partie principale (40) est à nouveau déployée, **en ce que** le premier et le second élément de la bande de partie principale (40) sont transportés en étant superposés aux segments latéraux respectifs et assemblés les uns aux autres.

5. Procédé selon la revendication 1, **caractérisé en ce que** les segments latéraux avant et arrière (20, 22) respectifs sont formés par différentes bandes de segments latéraux, du fait qu'au cours d'une première étape d'application, des segments latéraux arrière ou avant (20, 22) sont assemblés des deux côtés au cours d'un procédé de découpe et de placement à la bande de partie principale (40), et **en ce qu'**au cours d'une seconde étape d'application, une bande de segments latéraux (44) amenée en continu est assemblée des deux côtés à la bande de partie principale (40) précédemment repliée puis une opération de séparation reprenant les bandes de segments latéraux (44) est effectuée des deux côtés.

6. Procédé selon la revendication 5, **caractérisé en ce qu'**au cours de la première étape d'application, des segments latéraux arrière ou avant (20, 22) sont assemblés au cours d'un procédé de découpe et de placement à un premier élément de la bande de partie principale (40) et la bande de partie principale (40) est à nouveau déployée, et **en ce qu'**au cours d'une seconde étape d'application, une bande de segments latéraux (44) amenée sans fin est assemblée à un second élément précédemment replié de la bande de partie principale (40) puis une procédure de séparation reprenant les bandes de segments latéraux (44) est effectuée des deux côtés et la bande de partie principale (40) est à nouveau déployée, et **en ce que** le premier et le second élément de la bande de partie principale (40) sont transportés en étant superposés aux segments latéraux (20, 22) respectifs et assemblés les uns aux autres.

7. Procédé selon l'une quelconque des revendications précédentes, **caractérisé en ce qu'**une section libre (66) menant au bord intérieur (68) respectif de la bande de segments latéraux (44, 72) respectifs est effectuée dans la bande de segments latéraux (44, 72) respective, en plus de l'étape de séparation (52) mentionnée.

8. Procédé selon la revendication 7, **caractérisé en ce que** la section libre (66) est formée dans la bande de segments latéraux (44, 72) sans fin avant assemblage à la bande de partie principale (44).

9. Procédé selon la revendication 7 ou 8, **caractérisé en ce que** la section libre (66) présente une configuration en particulier sensiblement en forme de Y ou de V, s'élargissant à partir du bord intérieur (68) de la bande de segments latéraux (44, 72), ou une configuration d'agrafe cintrée à plat.

10. Procédé selon l'une quelconque des revendications précédentes, **caractérisé en ce que** la bande de partie principale (40) est guidée sur un seul corps rotatif (76) au cours du repliement, de l'assemblage aux bandes de segments latéraux (44, 72) et de la mise en oeuvre de la procédure de séparation (52).

11. Procédé selon l'une quelconque des revendications précédentes, **caractérisé en ce qu'**une courbe de vitesse respective de la bande de partie principale (40) destinée à la mise en oeuvre du repliement et/ou du déploiement est mise en oeuvre au moyen d'ensembles de rouleaux danseurs (58, 60) montés en amont et/ou montés en arrière.

12. Dispositif de fabrication d'une couche d'incontinence jetable (46) destiné à fabriquer une couche d'incontinence jetable selon l'une ou plusieurs des revendications précédentes, comprenant des dispositifs d'amenée (62) pour la bande de partie principale (40) et pour les bandes de segments latéraux (44, 72) bilatérales et comprenant un dispositif d'assemblage (48) destiné à fixer les bandes de segments latéraux (44, 72) aux zones de bord longitudinal (24) de la bande de partie principale (40) et comprenant un dispositif de découpage (50) destiné à définir le contour des segments latéraux arrière et/ou avant (20, 22), **caractérisé en ce qu'**un dispositif de repliement (75) pour la bande de partie principale (40) est disposé dans le sens machine (42) devant le dispositif d'assemblage (48) et devant le dispositif de découpage (50) et est destiné à replier la bande de partie principale (40) transversalement au plan de la bande et transversalement au sens machine (42).

13. Dispositif selon la revendication 12, **caractérisé en ce que** le dispositif de repliement (75), le dispositif d'assemblage (48) et le dispositif de découpage (50) sont réalisés ou disposés dans le même corps rotatif (76).

14. Dispositif selon la revendication 12 ou 13, **caractérisé en ce qu'**un ensemble de rouleaux danseurs (60, 58) est monté en avant et/ou en arrière du dispositif de repliement (75), du dispositif d'assemblage (48) et du dispositif de découpage (50) afin de réaliser la courbe de vitesse de la bande de partie principale (40) destinée à mettre en oeuvre le repliement et le déploiement.
